(19) Europäisches Patentamt / European Patent Office / Office européen des brevets

(11) **EP 4 385 428 A1**

(12) **EUROPEAN PATENT APPLICATION**

(43) Date of publication:
**19.06.2024 Bulletin 2024/25**

(21) Application number: **22212746.6**

(22) Date of filing: **12.12.2022**

(51) International Patent Classification (IPC):
**A61B 17/22** (2006.01)  **A61B 17/3207** (2006.01)
**A61F 2/24** (2006.01)

(52) Cooperative Patent Classification (CPC):
**A61B 17/2202; A61B 17/320725; A61F 2/2436;**
A61B 2017/22098; A61F 2/2418

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Aorticlab Srl**
**10010 Colleretto Giacosa (TO) (IT)**

(72) Inventors:
• **FERMI, Enrico**
**29122 Piacenza (IT)**

• **PASQUINO, Enrico**
**1073 Savigny (CH)**
• **BONETTI, Francesco**
**10122 Torino (IT)**
• **OSTA, Franco**
**15020 Mombello Monferrato (IT)**

(74) Representative: **Grosfillier, Philippe**
**André Roland SA**
**IP Attorneys & Services**
**Avenue Collonges 21**
**1004 Lausanne (CH)**

Remarks:
The references to the drawing no. 11 are deemed to be deleted (Rule 56(4)(6) EPC).

(54) **TRANSFEMORAL SYSTEM FOR THE LOCALIZED TREATMENT OF AORTIC VALVE STENOSIS**

(57)  A transfemoral system for the localized treatment of aortic valve stenosis comprising a working catheter (3) and at least one tool being configured to slidably move through the working catheter (3); characterized by the fact that said tool is an expandable stent (26) configured to allow an opening of an aortic native valve, the stent (26) furthermore comprising an ultrasonic transducer (25).

Fig. 24

EP 4 385 428 A1

## Description

### Field of invention

The present invention relates to the treatment of aortic valve stenosis and more precisely to the treatment of calcified and/or fibrotic aortic valve leaflets, for instance by ultrasound.

### Background

The early treatment of aortic stenosis, due to calcification of the valve leaflets or fibrosis, is essential to defer the timing of valve replacement operations (both thoracic or transcatheter), and to restore valve conditions in inoperable subjects.

Delaying the implantation of an aortic valve prosthesis, for a few years, allows to improve the patient's quality of life as well as cost savings, thanks to the prevention of secondary pathologies or complications related to the artificial valve implant. It also reduces the risk of having to resort to a subsequent valve-in-valve TAVR implant.

Some mechanical solutions have been proposed such as valvuloplasty, using a balloon catheter, or based on scoring of the calcific leaflets to create fractures in the calcific deposits.

Valvuloplasty presents potential risks such as accidental rupture of the cusps, while both techniques have the major limitation of a reduced duration of efficacy of the treatment, in reducing valve stenosis, due to a rapid recalcification, already starting from the first months following treatment.

Other proposed solutions are based on the use of ultrasound obtained with impulse signals, capable of fragmenting the calcific deposits with small shockwaves emitted in the immediate vicinity of the aortic valve cusps.

See for example patent application EP 3 685 772 A1 "Device for the treatment of the calcification of tissues".

All proposed systems use a catheter that is introduced from the femoral artery, until it reaches the valve in the aortic area.

The simultaneous presence, in the catheter, of electrical connection cables, insulation systems, guide wires, temporary artificial valves and any other structures, requires a necessary reduction in the dimensions of the active ablation elements to the detriment of the effectiveness of the treatment. The width of the surface of the ultrasound sources affects both the acoustic intensity of the ultrasound field and the size of the treated valve area.

### General description of the invention

The present invention provides a transfemoral system for the localized treatment of aortic valve stenosis comprising a working catheter and at least one tool being configured to slidably move through the working catheter. The system according to the invention advantageously comprises several tools that can be alternatively used within the same working catheter. Consequently, the dimensions of each tool may be maximized.

The invention more precisely concerns a transfemoral system, as defined in the claims, for the localized treatment of aortic valve stenosis

According to one embodiment of the invention, the system is characterized by the fact that at least one tool is an expandable stent configured to allow an opening of an aortic native valve. The stent furthermore contains at least one ultrasonic transducer.

In a general manner, the system according to the present invention includes:

- a first working catheter, for navigation, to reach the aortic site from the peripheral femoral artery. Preferably the working catheter includes an inflatable and retractable tip, and advantageously a guide wire, inserted through the tip;

- preferably several different tools tailored to the treatment of the specific problems that cause valve stenosis aspect of aortic valve stenosis, wherein each tool can be inserted into or retracted from the working catheter.

The following three tools can be used in the system of the present invention, only one of them, two or all three:

- A tool comprising ablation units based on rectangular piezoceramic transducers, for the fragmentation of intra-tissue calcium deposits or the treatment of valve fibrosis. As part of this tool, a particular support structure for these units allows them to be positioned precisely on the area of the leaflets to be treated, being able to rotate, flex and extend, keeping the distance between the transducers fixed, to create an emission surface equal to the sum of the surfaces of each single transducer. It also allows the transducers to follow the irregular profile of the valve leaflet that is thickened because of calcified deposits or due to fibrosis.
- Another tool based on mechanical percussion and vibration units for the specific treatment of fusion of the commissures.
- A third tool including an expandable stent which, once positioned in the patient's native valve, may gently force its opening. Preferably one or several cylindrical ultrasonic transducers are used. They support the opening action of the stenotic valve, breaking down calcium, if present, or remodelling the tissue if in presence of fibrosis.

Other tools may also be used in the system of the present invention.

The working catheter is preferably made of biocompatible material, for example polymeric, such as, but not limited to, polyamide, Grilamid or Vestamid, or equivalent materials, and has an external diameter of less than 18fr, or in any case not greater than 21fr.

**Detailed description of the invention**

The invention will be better understood in the present chapter, with some non-limiting examples illustrated by the following figures:

Figure 1 - Movement of the transcatheter system in the aortic arch

Figure 2 - Movement of the transcatheter system in the aortic arch, reaching the native valve site

Figure 3 - Deflated tip and retrieve through the navigation catheter which remains in place

Figure 4 - Tip with inflatable chamber and hypotube

Figure 5 - Sequence of device placement and retrieve leaving the navigation catheter in place

Figure 6 - Placement of one of the valvular treatment tools within the navigation catheter

Figure 7 - Variant of the tool with 3 strikers for the treatment of valve with fused or partially fused commissures

Figure 8 - Variant of the tool with a single striker for the treatment of valve with fused or partially fused commissures

Figure 9 - Placement of the 3-striker tool in the native aortic valve

Figure 10 - Placement of the single striker tool in the native aortic valve

Figure 11 - Simultaneous treatment of the commissures of the aortic valve

Figure 12 - Support structure for the ablation units

Figure 13 - Support structure for the ablation units with protective skirt

Figure 14 - Exploded view drawing of an ablation unit that comprises piezoceramic or polymer-based piezoelectric translator, fpc for electrical connection and isolation, metal support.

Figure 15 - Piezoceramic or polymer-based piezoelectric rectangular transducer mounted on metal support with fpc for electrical connection

Figure 16 - Ablation units mounted on a support structure

Figure 17 - Positioning of the ablation tool and temporary artificial valve

Figure 18 - Ablation unit and stent support structure

Figure 19 - Ablation unit support structure overlaid with the stent

Figure 20 - Expandable stent (without integrated artificial valve and without ultrasound emitters)

Figure 21 - Expandable stent with artificial valve

Figure 22 - Expandable stent with artificial valve and initial deployment of a "cylindrical ultrasound emitter (tube)"

Figure 23 - Expandable stent with artificial valve and final positioning of a "cylindrical ultrasound emitter (tube)"

Figure 24 - Expandable stent with artificial valve with one transducer outside the stent, open in the native valve: the structure is not expanded

Figure 25 - Expandable stent with artificial valve with one transducer outside the stent, open in the native valve: the structure is expanded

Figure 26 - Expandable stent with artificial valve with three transducers outside the stent, open in the native valve: the structure is not expanded

Figure 27 - Expandable stent with artificial valve with three transducers outside the stent, open in the native valve: the structure is expanded

Figure 28 - Expandable stent with artificial valve with one transducer inside the stent, open in the native valve: the structure is not expanded

Figure 29 - Expandable stent with artificial valve with one transducer inside the stent, open in the native valve: the structure is expanded

Figure 30 - Expandable stent with artificial valve with three transducers inside the stent, open in the native valve: the structure is not expanded

Figure 31 - Expandable stent with artificial valve with three transducers inside the stent, open in the native valve: the structure is expanded.

Numerical references used in the figures

1 Guidewire

2 Tip with inflatable/deflatable chamber

3 Working catheter

4 One-way blood valve

5 Hypotube

6 Inflatable chamber

7 Insufflation tube

8 Metal wedge

9 Linear Resonant Actuator (LRA)/vibration motor

10 Catheter coupling end

11 Strut connectors

12 Support cells

13 Tethering structures

14 Holes to tie the strands

15 Holes to fix transducer housing

16 Support legs

17 Struts in skirt region

18 Eyelet on strut

19 Central support

20 Protective flange

21 Ultrasound transducer

22 FPC flexible printed circuit

23 Transducer housing

24 Artificial valve

25 Piezoelectric tube (ultrasound emitter)

26 Expandable stent

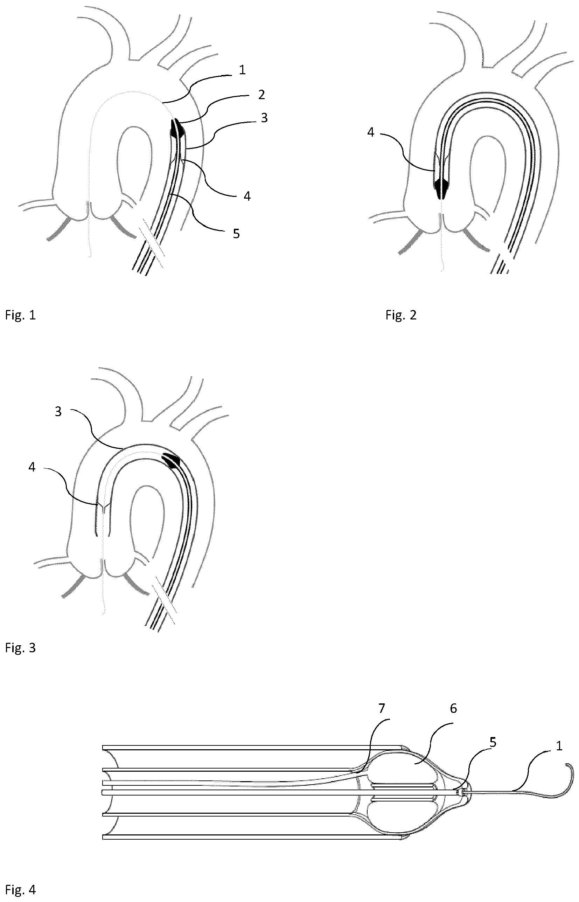

As shown in Figures 1, 2 and 3, the procedure for positioning the working catheter **3** involves the use of a guidewire **1** and the insertion of the device equipped with a tip **2** and hypotube **5** in which the guidewire **1** may slide.

According to one preferred embodiment of the present invention, the working catheter **3** is combined with a removable tip **2** made of inflatable, biocompatible plastic material, such as, but not limited to, silicone, polyurethane, polytetrafluoroethylene.

The tip **2,** which can be expanded by means of a liquid inserted via a syringe through an insufflation tube **7,** is inserted into the working catheter **3** and inflated, in the distal section of the catheter, until it is fully expanded, as shown in figure 4.

In this way, the tip **2** adheres perfectly to the walls of the catheter **3,** preventing blood from flowing into the catheter **3.**

The tip **2** and the working catheter **3** can be coated with a hydrophilic solution to facilitate tracking through artery, reducing friction with blood vessels.

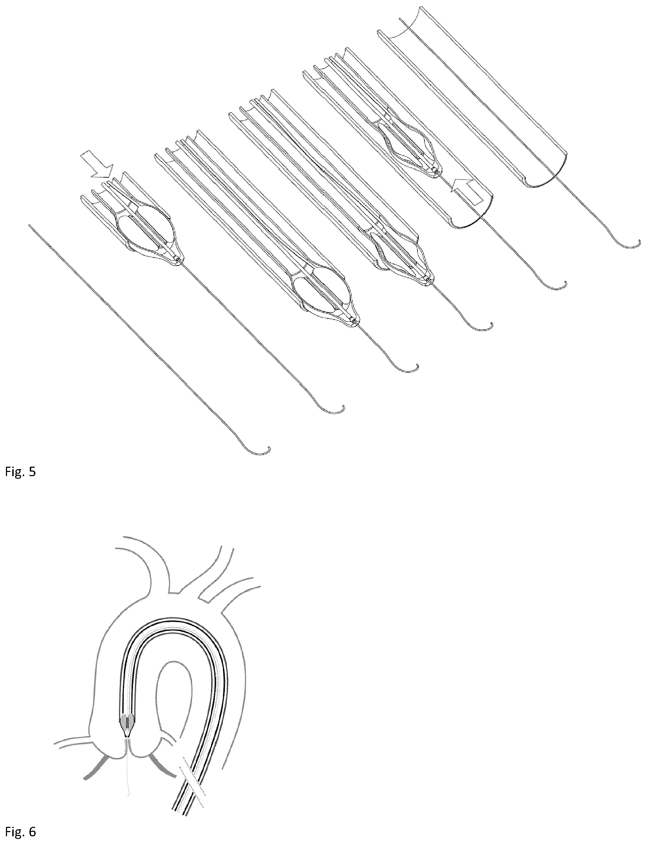

Once the aortic site is reached, as shown in figure 2, the tip chamber is deflated with a consequent reduction in the size of the tip **2,** which can be retracted and extracted from the working catheter **3,** as shown in Figure 3. The entire sequence is shown in figure 5.

Inside the working catheter **3,** a hemostatic valve **4,** for example a duckbill type or equivalent, prevents blood reflux when the tip **2** is retracted, waiting for the insertion of a tool within the working catheter **3.** See Fig. 3 and Fig. 6.

The three aortic valve stenosis treatment tools shown in those examples are of several different types of construction, the first two being dedicated to the treatment of calcified cusps and commissures and the third to the opening of the fused commissures because of calcification.

The embodiment of the first tool uses at least two piezoceramic or polymer-based piezoelectric transducers **21** for the emission of an ultrasonic field capable of fragmenting the calcium deposits in the valve cusps or modifying the structure of the cusps thanks to the microcavities induced by cavitation. This structural modification changes the pliability of the leaflets, which become more flexible.

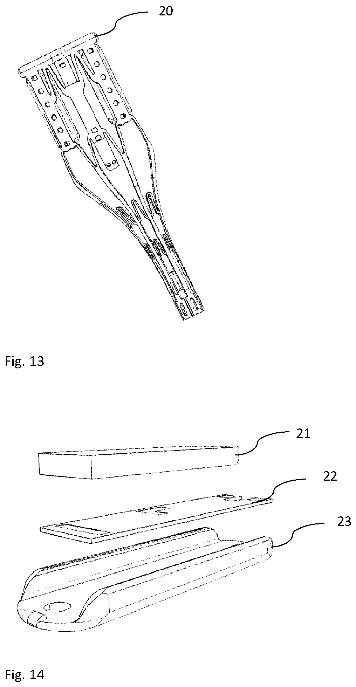

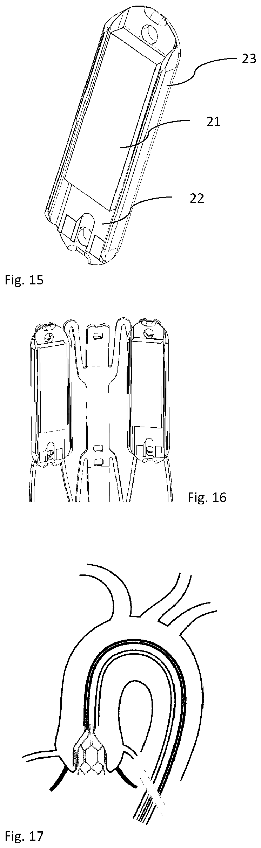

The transducers **21** are housed in metal supports **23** capable of safeguarding them from breaking and providing the backing function for focusing the emitted ultrasound field (Fig. 14 and 15).

The set composed of a piezoceramic or polymer-based piezoelectric transducer **21** and metal support **23** constitutes an ablation unit.

The electrical connection with the transducer terminals is obtained by means of a flexible FPC circuit **22** placed between the transducer housing **23** and the transducer **21**. This FPC **22** is made of polyamide with two exposed pads for electrical connection. The FPC **22** therefore also simultaneously provides electrical insulation, apart from the soldering pads, with the transducer **21.**

It is necessary to consider the acoustic impedance coupling effects of the transmitting transducer face and those due to the presence of the backing medium in contact with the parallel face behind it.

Inside the transducer **21,** the stress waves induced by the application of an electrical signal are generated at the center of the transducer 21 and propagate towards the faces that delimit it at the two ends.

The wave generated towards the *backing* interface is totally reflected towards the center of the transducer **21,** adding to the main wave. Ideally, the amplitude of the wave emitted by the transducer surface is exactly double that which would occur in the transmission medium without reflective *backing*.

If the emitting surface of the transducer **21** is coupled to the transmission medium, not directly, but through an appropriate layer, called *matching layer,* with thickness λ\4, the conditions of maximum emission are achieved (the acoustic impedance should be equal to the square root of the product between the characteristic acoustic impedance of the transducer **21** and the acoustic impedance of the medium, such as blood or water

$$Z_M = \sqrt{Z_p Z_T}$$

).

For applications where the transducer **21** must operate in an impulsive regime, it is necessary that the ultrasound pulse generated is made as short as possible, to avoid unwanted overlaps between direct impulse (in transmission) and reflected impulse (in reception).

Shorter pulses are obtained by applying a material with strong internal damping to the rear face. The acoustic impedance of the backing should be very similar to that of the transducer and to avoid loss of efficiency usually between 3 and 7 MRayl (e.g. resins loaded with metal powders and metal oxides, heraldite or epoxy cements).

To achieve good energy transmission from a normal piezoelectric transducer used for ultrasound generation, characterized by a high characteristic acoustic impedance, to biological tissues, which have a relatively low impedance, a layer of coupling medium is generally used (*matching layer*) of appropriate material, so-called "quarter wavelength(λ/4)".

For ablation units this layer corresponds to the thickness of the insulation resin layer.

The backing is instead obtained with a material of multiple thickness of λ/4. To obtain this effect, at the operating frequencies, the base of the metal supports of the piezoceramic transducers must be a few tenths of a millimeter.

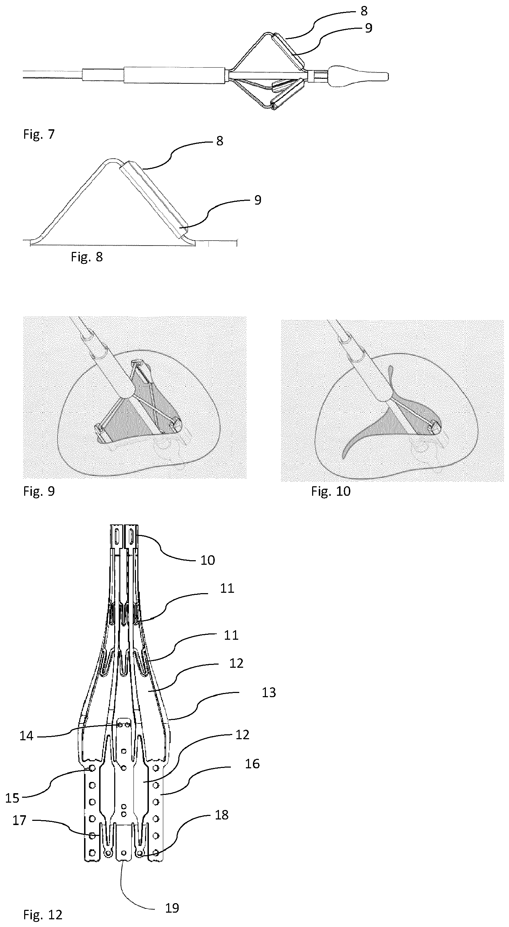

A peculiar metal structure (figure 12) provides support for the ablation units. This structure also serves as a mechanical constraint, as shown in Figure 12, so that the inter-distance between the transducers is predefined and kept fixed, to create an emission surface equal to the sum of the surfaces of each individual transducer. It also allows the transducers to follow the irregular profile of the valve leaflet that is thickened because of calcified deposits or due to fibrosis.

This structure (fig. 12) is made of metal alloy with shape memory effect (SME), such as eg. Nitinol NiTi and includes:

- a proximal tethering structure **13** with 4 arms with holes **10** for fixing to a hypotube **5** inside the vascular catheter;
- strut connectors **11** for connection between the arms to ensure adequate strength for the structure;
- two lateral support legs **16,** for fixing the ablation units (Fig. 15), comprising of transducer **21** + FPC **22** + metal support **23;**
- a central support **19** for housing a temperature sensor (for example a PT100 RTD, resistive, or a thermocouple). Some holes **14** on this central support **19** allow to fix elements such as tie wires made of steel or Kevlar or high grade polyester, ...) in order to flex or extend the entire structure during positioning on the native heart valve to be treated.

The terminal part of this structure (skirt region) includes two struts **17** with eyelets **18** for fixing a protective flange **20** made of polymer or other equivalent material. This protection saveguards the leaflets during the placement procedure of the ablation units.

This structure can rotate 360 ° allowing the control of the position of the ablation units with respect to the aortic valve to be treated.

The electrical connection is made via a flexible polyamide FPC **22** printed circuit with gold plated pads, soldered to the silver armature of the transducer with solder paste or conductive resin. The FPC also simultaneously provides electrical insulation with respect to the transducer support (made of metal and therefore conductive).

The multi-layer structure in figure 14, includes the piezoceramic or polymer-based piezoelectric transducer **21,**usually rectangular in shape, the metal support **23** and the electrical connection FPC **22** in the contact windows and at the same time for electrical insulation.

The electrical insulation of the entire ablation unit can be achieved through biocompatible coatings, such as Parylene-C or, preferably, polyurethane which allows good deformability of the piezoceramic or polymer-based piezoelectric transducers, excellent electrical insulation and mechanical resistance to avoid flaking as it slides into the catheter.

Through the working catheter **3** the ablation units reach the aortic valve site, where they emit impulsive ultrasonic fields at two frequencies, 3-4MHz and 100-200KHz.

These pulses have the form of low-power shock waves, with a positive peak followed by a negative one, with an amplitude about ¼ of the positive one, lasting about 20 - 60 ns.

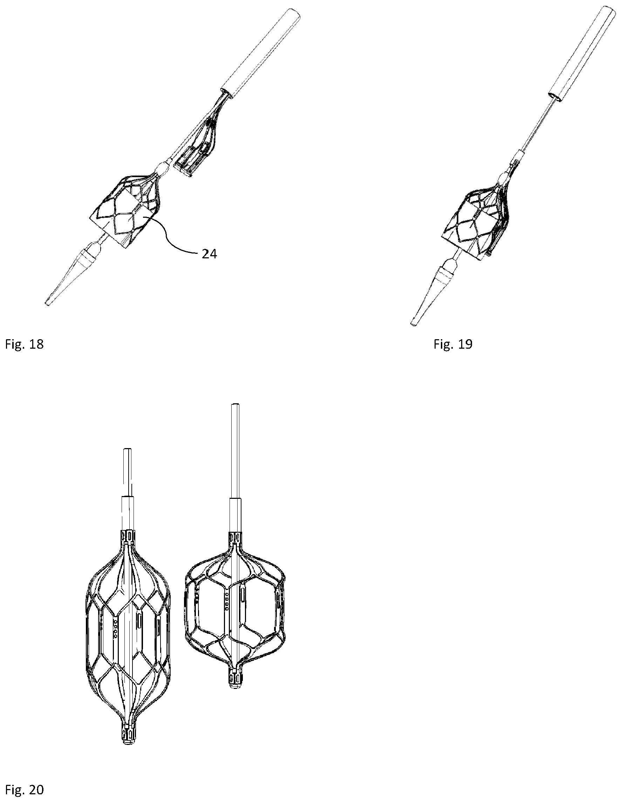

Once the aortic site is reached, the ablation units come out of the catheter **3** and open, taking the shape of a trident with the ablation units on the side arms as in figure 18, remaining side by side.

This configuration allows ultrasound to be delivered over a large portion of the calcific aortic valve leaflets, increasing the effectiveness of the treatment. The acoustic power, in fact, depends on the area of the ultrasound emission unit. Two piezoceramic transducers are kept side by side so increasing the emission surface.

The solution includes a temporary artificial valve **24** made of polymeric material or fabric dipped with polyester, for example, as already described in international patent application WO 2020/151995 A1. The treatment tool based on these transducers is positioned by placing the ablation units on the patient's native valve leaflets, held open by the artificial valve (Fig 19).

The solution also provides a specialized tool to treat the fused valve commissures (Fig. 7 and 8). The ablation unit of this third type includes a single-axis haptic vibration motor **9,** called LRA (Linear Resonant Actuator), to which a high hardness triangular wedge **8** is attached. Possible materials that can be used to make the triangular wedge are, for example: steel, titanium, ceramic.

This wedge-shaped striker has the function of a "chisel" able to open the commissures, in advance or even simultaneously, to the treatment with the ultrasound module.

The LRA motor is very compact in size, slightly smaller than the size of the delivery catheter.

This unit, comprising both the LRA motor and the triangular wedge, is positioned on an arm of a steel or nitinol structure which, when open, has the shape of a V, as shown in figure 8, while it is rectilinear when closed in the delivery catheter.

The catheter that includes this unit, reaches the aortic valve site, passing through the previously placed working catheter.

By retracting the covering sheath, the structure opens assuming an "inverted" V shape. The ablation unit, placed on the lower arm of this structure is positioned directly in the commissure to be treated (Fig. 10). The mechanical vibration generated by the motor and the consequent percussion imposed by the triangular wedge, promote the opening of the commissure partially closed or fused by calcification.

In a variant of the solution, three strikers are used, each positioned on an arm of the structure, as shown in Figure 7. This variant treats the three valve commissures simultaneously (Fig. 9).

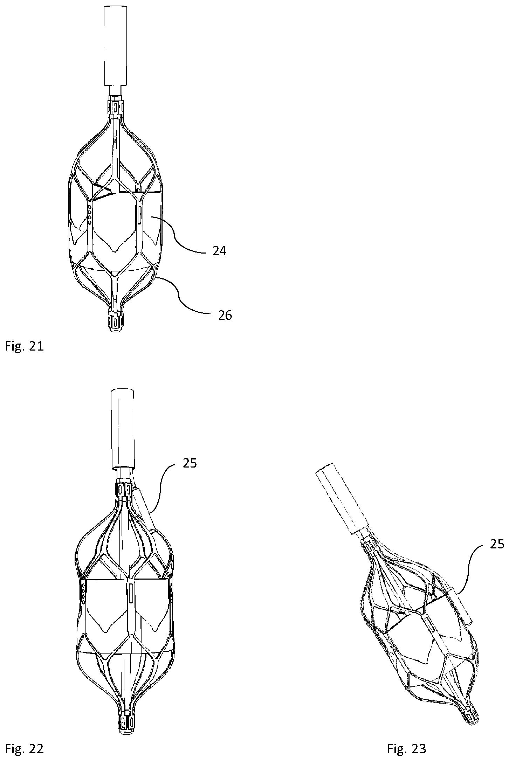

A third treatment tool for aortic valve stenosis includes the use of a stent **26** capable of expanding, (Fig. 20), once positioned in the patient's native valve to gently "force" the opening of the latter. In one embodiment, in addition to the mechanical action, the expandable stent tool includes at least one cylindrical piezoceramic transducer **25,** or tube-shaped, for the emission of the ultrasound field (Fig. 21). Compared to a valvuloplasty that has the risk of valve rupture, the expansion achieved by this instrument, is slow and controlled and supported by the combined action of ultrasounds that break down calcium, if present, or remodel the tissue if in the presence of fibrosis.

The choice of using a cylindrical "tube-shaped" transducer, for this configuration, allows to emit the ultrasound field radially, allowing its simultaneous action on the leaflets and on the commissures, even if of lower intensity than the rectangular transducers used for the ablation tools previously described. However, the intensity is sufficient if used as an integrated action to the mechanical expansion action of the stent **26**: the combination of gradual expansion and reshaping action of ultrasound increases the diameter of the native valve, reducing the obstruction of blood flow.

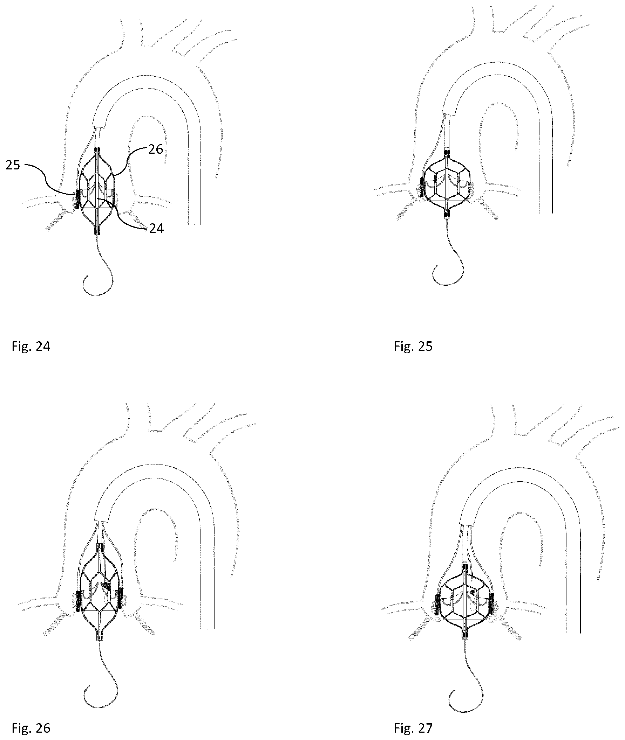

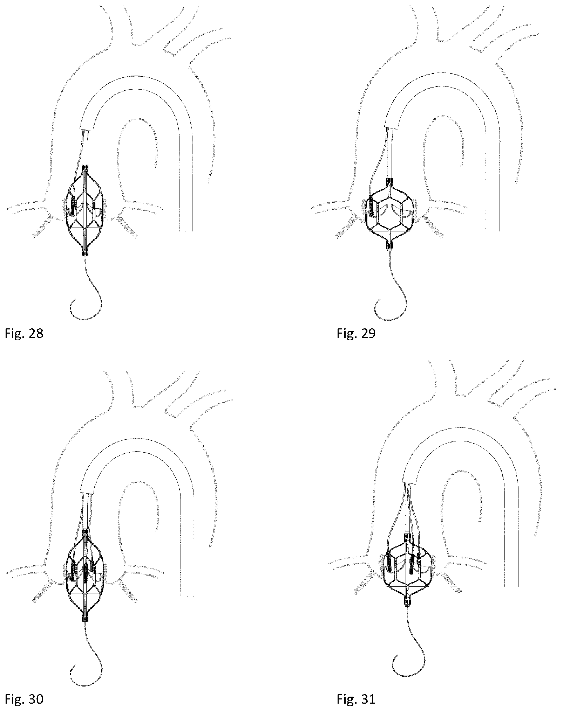

Variants of this structure include: the use of a transducer **25** external to the stent **26** (Fig. 24-25), the use of 3 transducers external to the stent **26** (Fig. 26-27), the use of a transducer internal to the stent and external to the artificial valve (Fig. 28-29), the use of three transducers internal to the expandable stent (Fig. 30-31) and external to the artificial valve.

**Claims**

1. A transfemoral system for the localized treatment of aortic valve stenosis comprising a working catheter (3) and at least one tool being configured to slidably move through the working catheter (3); **characterized by** the fact that said tool is an expandable stent (26) configured to allow an opening of an aortic native valve, the stent (26) furthermore comprising an ultrasonic transducer (25).

2. System according to claim 1 wherein the ultrasonic transducer (25) has a cylindrical shape.

3. System according to claim 1 or 2 wherein the stent (26) comprises several ultrasonic transducers (25).

4. System according to anyone of the previous claims wherein the ultrasonic transducer(s) (25) is/are external to the stent (26).

5. System according to anyone of the previous claims 1 to 3 wherein the ultrasonic transducer(s) (25) is/are internal to the stent (26).

6. System according to anyone of the previous claims wherein the stent (26) contains an artificial valve (24).

7. System according to anyone of the previous claims wherein the working catheter (3) includes an inflatable and retractable tip (2).

8. System according to anyone of the previous claims comprising another tool being configured to slidably move through the working catheter (3); **characterized by** the fact that said tool comprises a support structure and rectangular ultrasonic transducers (21) fixed to the support structure; said support structure being furthermore configured to directly position said rectangular ultrasonic transducers on valve leaflets to be treated.

9. System according to anyone of the previous claims comprising another tool being configured to slidably move through the working catheter (3); **characterized by** the fact that said tool comprises mechanical percussion and vibrations units that are configured for the treatment of valve commissures.

10. System according to claim 9 wherein each mechanical percussion and vibration unit comprises a Linear Resonant Actuator (9) equipped with a metal wedge (8) capable of opening valve commissures.

Fig. 1

Fig. 2

Fig. 3

Fig. 4

Fig. 5

Fig. 6

Fig. 7

Fig. 8

Fig. 9

Fig. 10

Fig. 12

20

Fig. 13

21

22

23

Fig. 14

Fig. 15

Fig. 16

Fig. 17

Fig. 18

24

Fig. 19

Fig. 20

Fig. 21

Fig. 22

Fig. 23

Fig. 24

Fig. 25

Fig. 26

Fig. 27

Fig. 28

Fig. 29

Fig. 30

Fig. 31

Europäisches
Patentamt

European
Patent Office

Office européen
des brevets

# EUROPEAN SEARCH REPORT

Application Number

EP 22 21 2746

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/061869 A1 (FERMI ENRICO [IT] ET AL) 3 March 2022 (2022-03-03) * paragraph [0075] – paragraph [0111]; figures 1–15 * | 1–10 | INV. A61B17/22 A61B17/3207 A61F2/24 |
| A | WO 2022/023968 A1 (OPCONSULTING S R L [IT]; MYRA MEDICAL S A R L [CH]; FERMI ENRICO [IT]) 3 February 2022 (2022-02-03) * page 19, line 8 – page 21, line 5; figures 6–8B, 12 * | 1,2,5 | |
| A | US 2011/264039 A1 (THIELEN JOSEPH MICHAEL [US] ET AL) 27 October 2011 (2011-10-27) * paragraph [0077]; figure 4c * | 1 | |
| A | US 2011/257562 A1 (SCHAER ALAN [US]) 20 October 2011 (2011-10-20) * paragraph [0086]; figure 5 * | 2 | |
| A | US 2022/096105 A1 (BENARY RAPHAEL [IL] ET AL) 31 March 2022 (2022-03-31) * page 6, line 28 – page 7, line 6; figures 6A,B * | 6 | TECHNICAL FIELDS SEARCHED (IPC) A61B A61F |
| A | US 2011/118634 A1 (GOLAN EREZ [IL]) 19 May 2011 (2011-05-19) * paragraph [0067] – paragraph [0071]; figures 13–16 * * column 100 – column 101; figures 40, 41 * | 9,10 | |
| A | US 2012/253358 A1 (GOLAN EREZ [IL]) 4 October 2012 (2012-10-04) * paragraph [0052] – paragraph [0055]; figures 16, 17 * | 9,10 | |

The present search report has been drawn up for all claims

| Place of search | Date of completion of the search | Examiner |
|---|---|---|
| The Hague | 13 April 2023 | Moers, Roelof |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                             

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 22 21 2746**

This annex lists the patent family members relating to the patent documents cited in the above-mentioned European search report.
The members are as contained in the European Patent Office EDP file on
The European Patent Office is in no way liable for these particulars which are merely given for the purpose of information.

**13-04-2023**

| Patent document cited in search report | | | Publication date | Patent family member(s) | | | Publication date |
|---|---|---|---|---|---|---|---|
| US 2022061869 | A1 | | 03-03-2022 | CA | 3125936 | A1 | 30-07-2020 |
| | | | | CN | 113490459 | A | 08-10-2021 |
| | | | | EP | 3685772 | A1 | 29-07-2020 |
| | | | | EP | 3914168 | A1 | 01-12-2021 |
| | | | | JP | 2022517779 | A | 10-03-2022 |
| | | | | US | 2022061869 | A1 | 03-03-2022 |
| | | | | WO | 2020151995 | A1 | 30-07-2020 |
| WO 2022023968 | A1 | | 03-02-2022 | NONE | | | |
| US 2011264039 | A1 | | 27-10-2011 | NONE | | | |
| US 2011257562 | A1 | | 20-10-2011 | NONE | | | |
| US 2022096105 | A1 | | 31-03-2022 | US | 2022096105 | A1 | 31-03-2022 |
| | | | | WO | 2018078563 | A1 | 03-05-2018 |
| US 2011118634 | A1 | | 19-05-2011 | EP | 2326264 | A2 | 01-06-2011 |
| | | | | ES | 2659322 | T3 | 14-03-2018 |
| | | | | JP | 5588978 | B2 | 10-09-2014 |
| | | | | JP | 2011528963 | A | 01-12-2011 |
| | | | | US | 2011118634 | A1 | 19-05-2011 |
| | | | | WO | 2010014515 | A2 | 04-02-2010 |
| US 2012253358 | A1 | | 04-10-2012 | CN | 102791207 | A | 21-11-2012 |
| | | | | EP | 2506781 | A1 | 10-10-2012 |
| | | | | US | 2012253358 | A1 | 04-10-2012 |
| | | | | US | 2021393281 | A1 | 23-12-2021 |
| | | | | WO | 2011069025 | A1 | 09-06-2011 |

EPO FORM P0459

For more details about this annex : see Official Journal of the European Patent Office, No. 12/82

**EP 4 385 428 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3685772 A1

- WO 2020151995 A1